# EUROPEAN PATENT APPLICATION

(11) **EP 4 394 046 A1**
(43) Date of publication of application: **03.07.2024**
(21) Application number: 22717337.4
(22) Date of filing: 24.02.2022
(51) Int. Cl.: C12Q 1/6806, C40B 50/06, C40B 40/06

(54) **METHOD FOR CONSTRUCTING RNA AND DNA NEXT-GENERATION SEQUENCING LIBRARY, AND NEXT-GENERATION SEQUENCING KIT**

(30) Priority: 25.08.2021 CN 202110983590
(71) Applicant: Daan Gene Co., Ltd., Guangzhou, Guangdong 510665 (CN)
(72) Inventor: JIANG, Xiwen, Guangzhou, Guangdong 510665 (CN); LIANG, Zhikun, Guangzhou, Guangdong 510665 (CN); ZHANG, Lishan, Guangzhou, Guangdong 510665 (CN); WU, Yilan, Guangzhou, Guangdong 510665 (CN)
(74) Representative: Handsome I.P. Ltd
(86) International application number: PCT/CN2022/077618
(87) International publication number: WO 2023/024465

(57) **Abstract**

The present disclosure relates to the field of biotechnology, and provides a method for constructing a second-generation sequencing library of RNA and DNA, and a second-generation sequencing kit. The method includes: performing first-strand synthesis on an RNA nucleic acid and a DNA nucleic acid to obtain a first-strand cDNA; performing second-strand synthesis on the first-strand cDNA to obtain a second-strand cDNA; obtaining an A-tailed product by fragmentation, end repair, phosphorylation, and A-tailing on the second-strand cDNA; performing adapter ligation and a first purification treatment on the A-tailed product to obtain a target RNA fragment and DNA fragment, and recovering the same; and carrying out a PCR amplification reaction on target RNA fragment and DNA fragment to construct and obtain the second-generation sequencing library of RNA and DNA. This method can construct the second-generation sequencing library by the whole library construction process, thereby simplifying the process steps, greatly reducing the operation process and experimental time, and minimizing the loss of nucleic acid samples. In addition, it is necessary to construct two libraries, which greatly saves manpower, reagents, time and other costs.

## Description

This application is based upon and claims priority to Chinese Patent Application No. 202110983590.4, filed before China National Intellectual Property Administration on August 25, 2021 and entitled "METHOD FOR CONSTRUCTING SECOND-GENERATION SEQUENCING LIBRARY OF RNA AND DNA, AND SECOND-GENERATION SEQUENCING KIT," the entire contents of which are incorporated herein by reference.

### TECHNICAL FIELD

The present application relates to the field of biotechnology, and in particular, relates to a method for constructing a second-generation sequencing library of RNA and DNA, and a second-generation sequencing kit.

### BACKGROUND

Currently, for samples infected by unknown pathogenic microorganisms in the market, most of the conventional schemes adopt the method of constructing an RNA library and a DNA library separately, which is time-consuming, laborious, and costly.

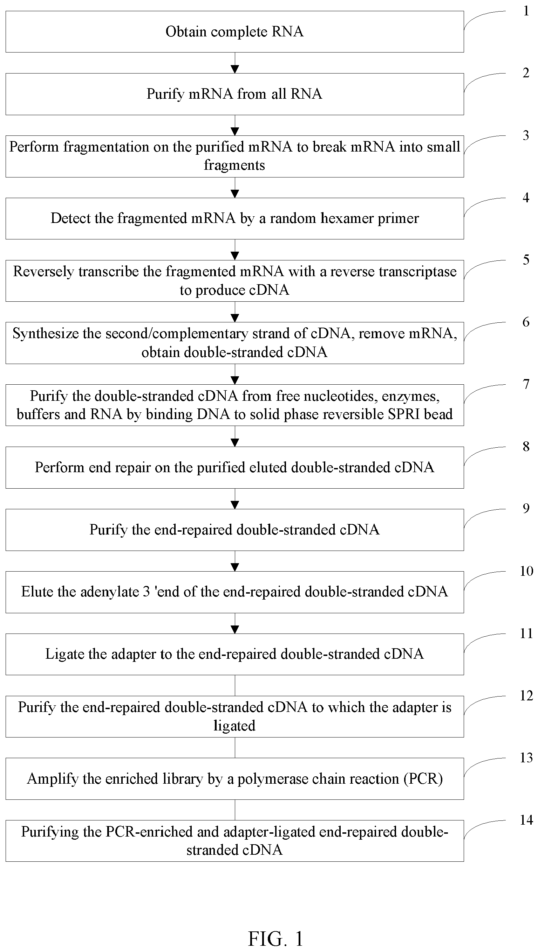

As illustrated in FIG. 1, a conventional construction process of an RNA library includes: 1, obtaining about 1 to 10 µg of pure and complete RNA after quality inspection; 2, purifying mRNA from all RNA; typically, this is done by annealing all of the RNA onto oligo-dT magnetic beads; wherein two rounds of purification may be performed to remove non-specifically bound ribosomes and other RNA from the oligo-dT; then releasing or separating the mRNA from the oligo-dT magnetic beads; 3, performing fragmentation on the purified mRNA with a fragmentation reagents to break mRNA strands into small fragments; 4, detecting the fragmented mRNA by a random hexamer primer; 5, reversely transcribing the fragmented mRNA with a reverse transcriptase to produce cDNA; 6, synthesizing the second/complementary strand of cDNA, and removing mRNA; wherein the product is double-stranded cDNA (ds cDNA); 7, purifying the double-stranded cDNA from free nucleotides, enzymes, buffers and RNA by binding DNA to solid phase reversible SPRI beads; 8, performing end repair on the purified eluted double-stranded cDNA; 9, purifying the end-repaired double-stranded cDNA; 10, eluting the adenylate 3 'end of the end-repaired double-stranded cDNA; 11, ligating the adapter to the end-repaired double-stranded cDNA; 12, purifying the end-repaired double-stranded cDNA to which the adapter is ligated; 13, amplifying the enriched library by a polymerase chain reaction (PCR); wherein the sequence from the adapter is used as a primer, a small number of cycles (12 to 16) are performed to amplify the existing sequence; 14, purifying the PCR-enriched and adapter-ligated end-repaired double-stranded cDNA.

It is apparent that in the above 14 steps, six steps are performed for purification. Obviously, the steps for constructing the existing RNA library are tedious, the total amount and quality of RNA are required to be high, and it takes a long time to purify the RNA with a plurality of magnetic beads. The products of two-chain synthesis and the products of adapter ligation of end-repaired products need to be purified, and the steps of end-repair and A-tailing cannot be integrated. These procedures are not only costly and time-consuming, but also multiple steps of purification inevitably increase the loss of RNA samples. However, RNA virus itself has low content and poor stability, which is seriously lost in the course of multi-round experimental operation, and is easy to be missed because it cannot reach the detection limit.

Therefore, it would be of great practical value to develop a simplified library construction method, in particular, a library co-construction method of RNA and DNA suitable for lower starting quantities and poor quality.

### SUMMARY

Based on the above-mentioned problems, one problem to be solved by the present disclosure is to provide a method for constructing a second-generation sequencing library of RNA and DNA, which is simple to construct a library, has a low starting amount, and is highly practical.

Another problem to be solved by the present disclosure is to provide a second-generation sequencing kit of RNA and DNA.

One technical solution of the present disclosure is as follows:
A method for constructing a second-generation sequencing library of RNA and DNA includes:
S1, performing first-strand synthesis on an RNA nucleic acid and a DNA nucleic acid to obtain a first-strand cDNA;
S2, performing second-strand synthesis on the first-strand cDNA to obtain a second-strand cDNA;
S3, obtaining an A-tailed product by fragmentation, end repair, phosphorylation, and A-tailing on the second-strand cDNA by a one-step reaction;
S4, performing adapter ligation and a first purification treatment on the A-tailed product to obtain a target RNA fragment and DNA fragment, and recovering the same; and
S5, carrying out a PCR amplification reaction using the recovered target RNA fragment and DNA fragment as a template to enrich the target RNA fragment and DNA fragment, and constructing and obtaining the second-generation sequencing library of RNA and DNA.

The present disclosure further provides a second-generation sequencing kit of RNA and DNA prepared by the method for constructing the library as described above.

With respect to the related art, the method for constructing the second-generation sequencing library of RNA and DNA has the following advantages:
(1) RNA and DNA library co-construction saves manpower, reagents, time and other costs, without constructing two libraries.
(2) The overall library construction process and steps are simplified, the operations and experimental time are greatly reduced, and the loss of nucleic acid samples may be minimized. Therefore, the requirements for the total amount and quality of DNA and RNA are lower, which can be applied to the detection of pathogenic microorganisms with lower initial amount and poor quality of RNA and DNA, thus greatly improving the detection rate.

### BRIEF DESCRIPTION OF THE DRAWINGS

For clearer descriptions of technical solutions according to the embodiments of the present disclosure, drawings that are to be referred for description of the embodiments are briefly described hereinafter. Apparently, the drawings described hereinafter merely illustrate some embodiments of the present disclosure. Persons of ordinary skill in the art may also derive other drawings based on the drawings described herein without any creative effort.
FIG. 1 is a flowchart of a conventional process for constructing a second-generation sequencing library of RNA; and
FIG. 2 is a flowchart of a process for constructing a second-generation sequencing library of RNA and DNA according to the present disclosure.

### DETAILED DESCRIPTION

Unless defined otherwise, all technical and scientific terms used herein have the same meaning as commonly understood by one of ordinary skill in the art to which this application pertains. The terms used herein in the specification of present disclosure are only intended to illustrate the specific embodiments of the present disclosure, instead of limiting the present disclosure. The terms "comprise," "include," and any variations thereof in the specification, claims, and the description of the drawings of the present disclosure are intended to cover a non-exclusive inclusion. The terms such as "first," "second," and the like in the specifications, claims or the accompanying drawings of the present invention are intended to distinguishing different objects but are not intended to define a specific sequence.

The term "embodiment" in this specification signifies that the specific characteristic, structures or features described with reference to the embodiments may be covered in at least one embodiment of the present disclosure. This term, when appears in various positions of the description, neither indicates the same embodiment, nor indicates an independent or optional embodiment that is exclusive of the other embodiments. A person skilled in the art would implicitly or explicitly understand that the embodiments described in this specification may be incorporated with other embodiments.

Some exemplary embodiments of the present disclosure are described in detail hereinafter with reference to the accompanying drawings.

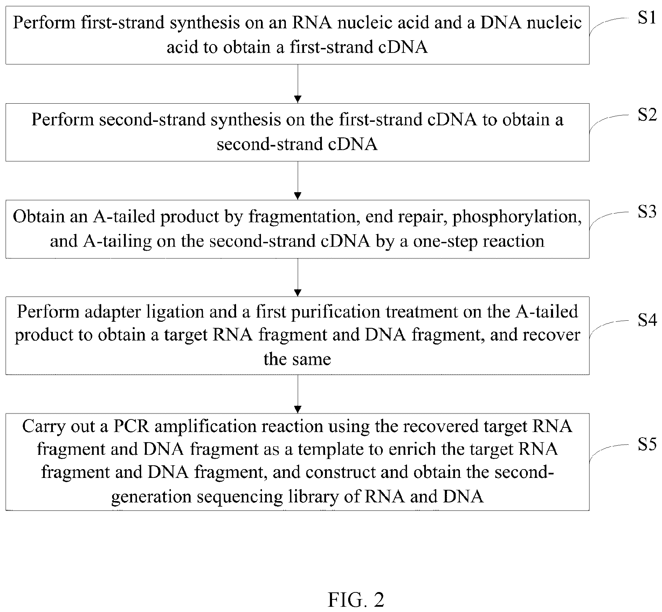

The present disclosure provides a process for constructing a second-generation sequencing library of RNA and DNA. As illustrated in FIG. 2, the process includes the following steps:
S1, performing first-strand synthesis on an RNA nucleic acid and a DNA nucleic acid to obtain a first-strand cDNA;
S2, performing second-strand synthesis on the first-strand cDNA to obtain a second-strand cDNA;
S3, obtaining an A-tailed product by fragmentation, end repair, phosphorylation, and A-tailing on the second-strand cDNA by a one-step reaction;
S4, performing adapter ligation and a first purification treatment on the A-tailed product to obtain a target RNA fragment and DNA fragment, and recovering the same; and
S5, carrying out a PCR amplification reaction using the recovered target RNA fragment and DNA fragment as a template to enrich the target RNA fragment and DNA fragment, and constructing and obtaining the second-generation sequencing library of RNA and DNA.

In the above process, further, in step S1, the first-strand synthesis further includes the following steps:
S11, performing a binding treatment on RNA and a random primer (100 µM) at 65°C for 5 min to obtain an RNA binding product; and
S12, mixing and reacting the RNA binding product with a reaction solution A, an enzyme B, and an enzyme C to obtain the first-strand cDNA; wherein the reaction solution A includes Tris-HCl (having a pH of 8.3) at a final concentration of 300 mM, MgCl₂ at a final concentration of 18 mM, KCl at a final concentration of 300 mM, DTT at a final concentration of 60 mM, dATP at a final concentration of 3 mM, dGTP at a final concentration of 3 mM, dCTP at a final concentration of 3 mM, and dTTP at a final concentration of 3 mM; the enzyme B is a reverse transcriptase (M-MMLV enzyme); and the enzyme C is a murine RNase inhibitor (RNasin) enzyme.

Preferably, in step S12, the mixing reaction is carried out under: a reaction temperature of 25°C and a reaction duration of 5 to 15 min, or a temperature of 42°C and a duration of 10 to 20 min, or a reaction temperature of 70°C and a reaction duration of 10 to 30 min.

In the above construction process, still further, in step S2, the second-strand synthesis includes the following steps:
mixing and reacting the first-strand cDNA with a reactant D and an enzyme mixed solution E to obtain the second-strand cDNA; wherein the reaction solution D includes Tris-HCl (having a pH of 8.3) at a final concentration of 100 mM, MgCl₂ at a final concentration of 6 mM, KCl at a final concentration of 300 mM, DTT at a final concentration of 20 mM, NAD+ at a final concentration of 208 µM, BSA at a final concentration of 400 µg/mL, dATP at a final concentration of 4 mM, dGTP at a final concentration of 4 mM, dCTP at a final concentration of 4 mM, and dTTP at a final concentration of 4 mM; and the enzyme mixed solution E includes a DNA polymerase I, an E. coli DNA ligase, and an RNase H.

Preferably, in the second-strand synthesis, the mixing reaction is carried out under a reaction temperature of 16°C and a reaction duration of 30 to 60 min.

In the above construction process, still further, in step S3, the one-step reaction includes the following steps:
mixing and reacting the second-strand cDNA with a reaction solution F and an enzyme mixed solution G to obtain the A-tailed product; wherein the reaction solution F includes Tris-HCl (having a pH of 7.5) at a final concentration of 200 mM, MgCl₂ at a final concentration of 100 mM, NaCl at a final concentration of 500 mM, DTT at a final concentration of 100 mM, Triton X-100 at a final concentration of 1.5%, ATP at a final concentration of 5 mM, dATP at a final concentration of 5 mM, dGTP at a final concentration of 5 mM, dCTP at a final concentration of 5 mM, and dTTP at a final concentration of 5 mM; and the enzyme mixed solution G is a mixed solution of a Vvn endonuclease mutan, a T7 endonuclease mutant, and a Taq DNA polymerase.

Preferably, the one-step reaction is carried out under: a reaction temperature of 37°C and a reaction duration of 10 to 30 min, or a reaction temperature of 65°C and a reaction duration of 20 to 30 min.

Further, in step S4, the adapter ligation includes the following steps:
mixing and reacting the A-tailed product with a reaction solution H, a reaction solution I, and an enzyme J to obtain an RNA fragment and a DNA fragment; wherein the reaction solution H includes Tris-HCl (having a pH of 7.5) at a final concentration of 75 mM, MgCl₂ at a final concentration of 15 mM, DTT at a final concentration of 15 mM, ATP at a final concentration of 2.75 mM, and PEG 8000 at a final concentration of 20 v/v%; and
the reaction solution I, depending on different sequencing platforms, may be an Illumina adapter or an MGISEQ adapter Ad153, at a final concentration of 2 µM; and the enzyme J is a T4 DNA ligase;

Preferably, a reaction of the adapter ligation is carried out under a reaction temperature of 20°C and a reaction duration of 30 min.

Preferably, in step S4, upon completion the adapter ligation reaction, the further purification treatment needs to be performed for a product obtained by the adapter ligation, which specifically includes the following steps:
S41, loading the RNA fragment and DNA fragment and 80 µL of magnetic beads into a centrifuge tube, and inverting or vortexing the centrifuge tube to sufficiently mix the RNA fragment and DNA fragment with the magnetic beads;
S42, repeatedly blowing and beating the RNA fragment and DNA fragment with an EP pipette to mix them well with the magnetic beads, and placing the centrifuge tube on a magnetic rack and standing at room temperature;
S43, removing a supernatant after the solution in the centrifuge tube is cleared;
S44, adding 200 µL of freshly prepared 80 v/v% ethanol to the centrifuge tube to rinse the magnetic beads;
S45, repeating steps S42 to S44;
S46, upon centrifuging the centrifuge tube, placing the centrifuge tube on the magnetic rack, and completely removing the supernatant in the centrifuge tube; and
S47, taking out a sample from the centrifuge tube, adding 22 µL of nuclease-removed ultrapure water, gently pipetting, beating and mixing well with a pipette, standing at room temperature for 2 min, then placing the same on the magnetic rack, and after the solution is cleared, obtaining the target RNA fragment and DNA fragment.

In the above construction process, still further, in step S5, the PCR amplification reaction includes the following steps:
mixing and reacting the target RNA fragment and DNA fragment obtained upon the first purification treatment with a reaction solution K and a reaction solution L to obtain the second-generation sequencing library of RNA and DNA; wherein the reaction solution K includes a 5*Q5 buffer at a final concentration of 80%, dATP at a final concentration of 0.8 mM, dGTP at a final concentration of 0.8 mM, dCTP at a final concentration of 0.8 mM, dTTP at a final concentration of 0.8 mM, and a Q5 hot-start ultra-fidelity DNA polymerase at a final concentration of 4%; and the reaction solution L, depending on different sequencing platforms, may be an Illumina library amplification primer or an MGISEQ library preparation primer, at a concentration of 15 µM.

Preferably, the PCR amplification reaction includes the following reaction stages under different reaction temperatures and reaction durations:
1) pre-denaturation stage: under a reaction temperature of 95°C and a reaction duration of 3 min;
2) denaturation stage: under a reaction temperature of 95°C and a reaction duration of 20s;
3) annealing stage: under a reaction temperature of 58°C and a reaction duration of 20s;
4) extension stage: under a reaction temperature of 72°C and a reaction duration of 20s;
5) final extension stage: under a reaction temperature of 72°C and a reaction duration of 5 min; and
6) storage stage: under a reaction temperature of 4°C;
wherein the stages 2) to 4) are separately performed for 10 to 14 cycles; and stages 1) and 5) are separately performed for one cycle.

Preferably, upon completion of the PCR amplification reaction, the method further includes a second purification treatment on a PCR amplification product:
S51, loading the PCR amplification product and 85 µL of magnetic beads into a centrifuge tube, and inverting or vortexing the centrifuge tube to sufficiently mix the PCR amplification product and the magnetic beads;
S52, repeatedly blowing and beating the PCR amplification product with an EP pipette to mix the amplification product with the magnetic beads sufficiently, and placing the centrifuge tube on a magnetic rack for standing at room temperature;
S53, removing a supernatant after the solution in the centrifuge tube is cleared;
S54, adding 200 µL of freshly prepared 80 v/v% ethanol to the centrifuge tube to rinse the magnetic beads;
S55, repeating steps S52 to S54;
S56, upon centrifuging the centrifuge tube, placing the centrifuge tube on the magnetic rack, and completely removing the supernatant in the centrifuge tube;
S57, taking out a sample from the centrifuge tube, adding 22 µL of nuclease-removed ultrapure water, gently pipetting, beating and mixing well with a pipette, standing at room temperature for 2 min, then placing the same on the magnetic rack, and after the solution is cleared, obtaining the second-generation sequencing library of RNA and DNA.

The present disclosure further provides a second-generation sequencing kit of RNA and DNA prepared by the method for constructing the library as described above. The sequencing kit is a high-throughput sequencing kit, also referred to as a next-generation sequencing (NGS) kit.

The construction process of the second-generation sequencing library of RNA and DNA is described in detail hereinafter with specific examples.

### 1. First-strand synthesis

1.1 The random primers (100 µM) were taken out from a -20°C refrigerator, and thawed at room temperature, and the following system was prepared in a PCR tube as listed in Table 1.

**Table 1 Components of nucleic acid samples in one-strand synthesis**

| Component | Volume (µL) |
|---|---|
| Nucleic acid samples (i.e., RNA and DNA nucleic acid samples) | 19 |
| Random primers | 1 |
| Total | 20 |

The reaction components were well mixed in the PCR tube by gentle vortex and then briefly centrifuged in a centrifuge for 3s.

1.2 The PCR tube was placed in a PCR instrument and the following reaction procedure was set, as listed in Table 2.

**Table 2 Configuration conditions of nucleic acid samples in first-strand synthesis**

| Temperature | Duration |
|---|---|
| Hot cover | 75°C |
| 65°C | 5 min |
| 4°C | Hold |

1.3 The reaction solution A, the enzyme B, and the enzyme C were taken out from the refrigerator at -20°C; the reaction solution A was thawed at room temperature, and inverted and mixed well for short-term centrifugation for future use; the enzyme B and enzyme C were thawed on ice to a liquid state, inverted and mixed well for short-term centrifugation, and placed on an ice box for future use. The following reaction system was prepared in a PCR tube as listed in Table 3.

**Table 3 Components of reaction system of first-strand synthesis**

| Component | Volume (µL) |
|---|---|
| Product | 20 |
| Reaction solution A | 5 |
| Enzyme B | 3 |
| Enzyme C | 0.5 |
| Nuclease-free water | 1.5 |
| Total | 30 |

The reaction solution A includes Tris-HCl (having a pH of 8.3) at a final concentration of 300 mM, MgCl₂ at a final concentration of 18 mM, KCl at a final concentration of 300 mM, DTT at a final concentration of 60 mM, dATP at a final concentration of 3 mM, dGTP at a final concentration of 3 mM, dCTP at a final concentration of 3 mM, and dTTP at a final concentration of 3 mM; the enzyme B is a reverse transcriptase (M-MMLV enzyme); and the enzyme C is a murine RNase inhibitor (RNasin) enzyme.

1.4 The reaction components were well mixed in the PCR tube by gentle vortex and then briefly centrifuged in a centrifuge for 3s.

1.5 The PCR tube was placed in a PCR instrument and the following reaction procedure was set, as listed in Table 4.

**Table 4 Reaction conditions of first-strand synthesis**

| Temperature | Duration |
|---|---|
| Hot cover | 10°C |
| 25°C | 10 min |
| 42°C | 15 min |
| 70°C | 15 min |
| 4°C | Hold |

In Table 4, the reaction conditions may be as follows: a reaction temperature of 25°C and a reaction duration of 10 min, or a reaction temperature of 42°C and a reaction duration of 15 min, or a reaction temperature of 70°C and a reaction duration of 15 min

### 2. Second-strand synthesis

2.1 The reaction solution D and the enzyme mixed solution E were taken out from the refrigerator at -20°C; the reaction solution D was thawed at room temperature, and inverted and mixed well for short-term centrifugation for future use; the enzyme mixed solution E was thawed on ice to a liquid state, inverted and mixed well for short-term centrifugation, and placed on an ice box for future use. The following reaction system was prepared in a PCR tube as listed in Table 5.

**Table 5 Components of reaction system of second-strand synthesis**

| Component | Volume (µL) |
|---|---|
| First-strand product (i.e., first-strand cDNA) | 30 |
| Reaction solution D | 5 |
| Enzyme mixed solution E | 5 |
| Total volume | 40 |

The reaction solution D includes Tris-HCl (having a pH of 8.3) at a final concentration of 100 mM, MgCl₂ at a final concentration of 6 mM, KCl at a final concentration of 300 mM, DTT at a final concentration of 20 mM, NAD+ at a final concentration of 208 µM, BSA at a final concentration of 400 µg/mL, dATP at a final concentration of 4 mM, dGTP at a final concentration of 4 mM, dCTP at a final concentration of 4 mM, and dTTP at a final concentration of 4 mM; and the enzyme mixed solution E includes a DNA polymerase I, an E. coli DNA ligase, and an RNase H.

2.2 The reaction components were well mixed in the PCR tube by gentle vortex and then briefly centrifuged in a centrifuge for 3s.

2.3 The PCR tube was placed in a PCR instrument and the following reaction procedure was set, as listed in Table 6.

**Table 6 Reaction conditions of second-strand synthesis**

| Temperature | Duration |
|---|---|
| Hot cover | Off |
| 16°C | 30 min |
| 4°C | Hold ≤ 1 h |

### 3. Fragmentation, end repair, phosphorylation, and A-tailing

3.1 The reaction solution F and the enzyme mixed solution G were taken out from the refrigerator at -20°C; the reaction solution F was thawed at room temperature, and inverted and mixed well for short-term centrifugation for future use; the enzyme mixed solution G was thawed on ice, inverted and mixed well for short-term centrifugation, and placed on an ice box for future use. The following reaction system was prepared in a PCR tube as listed in Table 7.

**Table 7 Components of one-step reaction system**

| Component | Volume (µL) |
|---|---|
| Second-strand product (i.e., second-strand cDNA) | 40 |
| Reaction solution F | 5 |
| Enzyme mixed solution G | 5 |
| Total | 50 |

The reaction solution F includes Tris-HCl (having a pH of 7.5) at a final concentration of 200 mM, MgCl₂ at a final concentration of 100 mM, NaCl at a final concentration of 500 mM, DTT at a final concentration of 100 mM, Triton X-100 at a final concentration of 1.5%, ATP at a final concentration of 5 mM, dATP at a final concentration of 5 mM, dGTP at a final concentration of 5 mM, dCTP at a final concentration of 5 mM, and dTTP at a final concentration of 5 mM; and the enzyme mixed solution G is a mixed solution of a Vvn endonuclease mutan, a T7 endonuclease mutant, and a Taq DNA polymerase.

3.2 The reaction components were well mixed in the PCR tube by gentle vortex and then briefly centrifuged in a centrifuge for 3s.

3.3 The PCR tube was placed in a PCR instrument and the following reaction procedure was set, as listed in Table 8.

**Table 8 Conditions of one-step reaction**

| Steps | Temperature | Duration |
|---|---|---|
| Hot cover 80°C | | On |
| 1 | 37°C | See the following table |
| 2 | 65°C | 20 min |
| 3 | 4°C | Hold |

The fragmentation duration needs to be determined according to the mass of input nucleic acid and the size of a target insert fragment. As listed in Table 9, the corresponding fragmentation duration is determined according to an expected size of an insert fragment of DNA. For example, in the case that a 100 bp DNA fragment needs to be inserted, the fragmentation duration is 25 to 30 min.

**Table 9 Reaction conditions of DNA fragmentation in one-step reaction system**

| Expected size of inserted fragment | Fragmentation time |
|---|---|
| 100 bp | 25 to 30 min |
| 150 bp | 15 to 25 min |
| 200 to 700 bp | 10 to 15 min |

Note: The recommended durations in Table 9 are measured using high quality Hela cell gDNA as a template. When the high-quality Hela cell gDNA is used for library construction, the distribution range of fragmentation products does not vary greatly (the distribution range is basically the same, but the positions of main peaks may be slightly different) under the same reaction duration with different input amounts. In the case that the quality of the Input DNA is poor or the fragmentation size is not within an expected range, it is recommended to adjust the fragmentation duration up or down by an amplitude of 2 to 5 min.

### 3.4 Adapter ligation

4.1 The reaction solution H and the reaction solution I were taken out from the refrigerator at -20°C, thawed at room temperature, mixed well and subjected to short-time centrifugation for future use; the enzyme J was taken out from the refrigerator at -20°C, centrifuged briefly and placed on an ice box for future use.

4.2 The following components were added sequentially to the fragmented, end-repaired and A-tailed product, as listed in Table 10.

**Table 10 Components of adapter ligation reaction of DNA fragments**

| Component | Volume (µL) |
|---|---|
| Product of previous step (i.e., A-tailed product) | 50 |
| Reaction solution H | 20 |
| Reaction solution I | 5 |
| Enzyme J | 5 |
| Total | 80 |

The reaction solution H includes Tris-HCl (having a pH of 7.5) at a final concentration of 75 mM, MgCl₂ at a final concentration of 15 mM, DTT at a final concentration of 15 mM, ATP at a final concentration of 2.75 mM, and PEG 8000 at a final concentration of 20 v/v%.

The reaction solution I, depending on different sequencing platforms, may be an Illumina adapter or an MGISEQ adapter Ad153, at a final concentration of 2 µM; and the enzyme J is a T4 DNA ligase.

4.3 The reaction components were well mixed in the PCR tube by gentle vortex and then briefly centrifuged in a centrifuge for 3s.

4.4 The PCR tube was placed in a PCR instrument and the following reaction procedure was set, as listed in Table 11.

**Table 11 Conditions of adapter ligation reaction of DNA fragments**

| Steps | Temperature | Duration |
|---|---|---|
| Hot cover | | Off |
| 1 | 20°C | 30 min |
| 2 | 4°C | Hold |

### 5 First purification of ligation product

5.1 The DNA purification magnetic beads were taken out from the refrigerator at 2 to 8°C, 30 min ahead, and stood still to allow their temperature to come to room temperature.

5.2 The DNA purification magnetic beads were well mixed by inverting or vortexing, 80 µL of magnetic beads were dispersed into a 1.5 mL low-adsorption centrifuge tube.

5.3 80 µL of the ligation product in the previous step was added to the magnetic beads and repeatedly blown and shaken well (the tip shall not leave the surface of the solution when mixing well, so as to prevent the generation of excessive bubbles).

5.4 The resulted solution was incubated for 5 min at room temperature to allow the DNA to bind to the magnetic beads.

5.5 The sample was placed on a magnetic rack, and after the solution was cleared (about 5 min), the supernatant was carefully removed.

5.6 The sample was always kept on the magnetic rack, 200 µL of freshly prepared 80% ethanol was added to rinse the magnetic beads, the resulted solution was incubated for 30s at room temperature, and then the supernatant was carefully removed.

5.7 Steps 5.3, 5.5, and 5.6 were repeated once for a total of two rinses.

5.8 The sample was instantly centrifuged for 5s, the sample was placed on the magnetic rack, the residual supernatant was completely removed with a 10µL pipette, the sample was always kept on the magnetic rack, and the magnetic beads were uncovered and dried at room temperature for about 5 to 10 min, until there was no reflection on the surface of magnetic beads.

5.9 The sample was taken out from the magnetic rack, 22 µL of nuclease-removed ultrapure water was added, the resulted solution was gently sucked and mixed well with a pipette, the solution was stood at room temperature for 2 min and then placed on the magnetic rack. After the solution was cleared (about 5 min), 20 µL of the supernatant was carefully pipetted into the PCR tube for the next reaction.

### 3.6 Library amplification

6.1 The reaction solution K and the reaction solution L were taken out from the refrigerator at -20 °C, the reaction solution K was placed on ice to thaw for 20 min, the reaction solution L was thawed at room temperature for 20 min, mixed well for short-term centrifugation for future use, and the following components were added sequentially to the ligation product purified in the previous step, as listed in Table 12.

**Table 12 Components of PCR amplification of library of RNA and DNA fragments**

| Component | Volume (µL) |
|---|---|
| Ligation product | 20 |
| Reaction solution K | 25 |
| Reaction solution L | 5 |
| Total | 50 |

6.2 The reaction components were well mixed in the PCR tube by gentle vortex and then briefly centrifuged in a centrifuge for 3s.

6.3 Amplifications were performed in a thermocycler according to the following procedure, as listed in Table 13.

**Table 13 Conditions of PCR amplification reaction of library of RNA and DNA fragments**

| Steps | Temperat ure | Duration | Number of cycles |
|---|---|---|---|
| Hot cover 105°C | | On | |
| Predenaturation | 95°C | 3 min | 1 |
| Denaturalizatio n | 95°C | 20s | X |
| Annealing | 58°C | 20s | |
| Extending | 72°C | 20s | |
| Totally extending | 72°C | 5 min | 1 |
| Storage | 4°C | Hold | |

Note: The input amount of nucleic acid in the constructed library was less than 10 ng, X = 14; the input amount of nucleic acid was 10 to 30 ng, X = 12; the input amount of nucleic acid was 30 to 50 ng, X = 10.

### 7 Second purification of amplified product fragments

7.1 The DNA purification magnetic beads were taken out from the refrigerator at 2 to 8°C, 30 min ahead, and stood still to allow their temperature to come to room temperature.

7.2 The DNA purification magnetic beads were well mixed by inverting or vortexing, 85 µL of DNA purification magnetic beads were dispersed into a 1.5 mL low-adsorption centrifuge tube.

7.3 Water was supplemented to 50 µL of the PCR product in the previous step to 100 µL, and then added to the magnetic beads and repeatedly blown and shaken well (the tip shall not leave the surface of the solution when mixing well, so as to prevent the generation of excessive bubbles).

7.4 The resulted solution was incubated for 5 min at room temperature to allow the DNA to bind to the magnetic beads.

7.5 The sample was placed on a magnetic rack, and after the solution was cleared (about 5 min), the supernatant was taken into a low adsorption centrifuge tube containing 40 µL DNA purification magnetic beads.

7.6 The resulted solution was incubated for 5 min at room temperature to allow the DNA to bind to the magnetic beads.

7.7 The sample was placed on a magnetic rack, and after the solution was cleared (about 5min), the supernatant was carefully removed.

7.8 The sample was always kept on the magnetic rack, 200 µL of freshly prepared 80% ethanol was added to rinse the magnetic beads, the resulted solution was incubated for 30s at room temperature, and then the supernatant was carefully removed.

7.9 Steps 7.3, 7.7, and 7.8 were repeated once for a total of two rinses.

7.10 The sample was instantly centrifuged for 5s, the sample was placed on the magnetic rack, the residual supernatant was completely removed with a 10µL pipette, the sample was always kept on the magnetic rack, and the magnetic beads were uncovered and dried at room temperature for about 5 to 10 min, until there was no reflection on the surface of magnetic beads.

7.11 The sample was taken out from the magnetic rack, 22 µL of nuclease-removed ultrapure water was added, the resulted solution was gently sucked and mixed well with a pipette, the solution was stood at room temperature for 2 min and then placed on the magnetic rack. After the solution was cleared (about 5 min), 20 µL of the supernatant was carefully pipetted into another 1.5 mL EP tube.

In another embodiment, a high-throughput second-generation sequencing kit of RNA and DNA is constructed. The sequencing kit includes the library of RNA and DNA constructed and obtained by the method as described above.

It is apparent that the embodiments described above are only exemplary ones, but not all embodiments of the present disclosure, and that the attached drawings illustrate exemplary embodiments of the present disclosure but do not limit the scope of the present disclosure. The present disclosure may be embodied in many different forms and, on the contrary, these embodiments are provided such that the present disclosures are thoroughly and completely understood. Although the present disclosure has been described in detail with reference to the above embodiments, those skilled in the art will be able to make modifications to the technical solutions disclosed in the specific embodiments or make equivalent substitutions for some of the technical features. Where an equivalent structure made by using the contents of the specification and the drawings of the present disclosure is directly or indirectly applied to other relevant technical fields, it is likewise within the scope of protection of the present disclosure.

## Claims

1. A method for constructing a second-generation sequencing library of RNA and DNA, comprising:
S1, performing first-strand synthesis on an RNA nucleic acid and a DNA nucleic acid to obtain a first-strand cDNA;
S2, performing second-strand synthesis on the first-strand cDNA to obtain a second-strand cDNA;
S3, obtaining an A-tailed product by fragmentation, end repair, phosphorylation, and A-tailing on the second-strand cDNA by a one-step reaction;
S4, performing adapter ligation and a first purification treatment on the A-tailed product to obtain a target RNA fragment and DNA fragment, and recovering the same; and
S5, carrying out a PCR amplification reaction using the recovered target RNA fragment and DNA fragment as a template to enrich the target RNA fragment and DNA fragment, and constructing and obtaining the second-generation sequencing library of RNA and DNA.

2. The method according to claim 1, wherein in step S1, the first-strand synthesis comprises:
S11, performing a binding treatment on RNA and a random primer at 65°C for 5 min to obtain an RNA binding product; and
S12, mixing and reacting the RNA binding product with a reaction solution A, an enzyme B, and an enzyme C to obtain the first-strand cDNA; wherein the reaction solution A comprises Tris-HCl, MgCl₂, KCl, DTT, dATP, dGTP, dCTP, and dTTP; the enzyme B is an M-MMLV enzyme; and the enzyme C is an RNasin enzyme.

3. The method according to claim 2, wherein in step S12, the mixing reaction is carried out under: a reaction temperature of 25°C and a reaction duration of 5 to 15 min, or a reaction temperature of 42°C and a reaction duration of 10 to 20 min, or a reaction temperature of 70°C and a reaction duration 10 to 30 min.

4. The method according to claim 1, wherein, in step S2, the second-strand synthesis comprises:
carrying out a second-strand synthesis reaction on the first-strand cDNA with a reactant D and an enzyme mixed solution E to obtain the second-strand cDNA; wherein the reaction solution D comprises Tris-HCl, MgCl₂, KCl, DTT, NAD+, BSA, dATP, dGTP, dCTP, and dTTP; and the enzyme mixed solution E comprises a DNA polymerase I, an E. coli DNA ligase, and an RNase H.

5. The method according to claim 4, wherein the second-strand synthesis reaction is carried out under a reaction temperature of 16°C and a reaction duration of 30 to 60 min.

6. The method according to claim 1, wherein, in step S3, the one-step reaction comprises:
mixing and reacting the second-strand cDNA with a reaction solution F and an enzyme mixed solution G to obtain the A-tailed product; wherein the reaction solution F comprises Tris-HCl, MgCl₂, NaCl, DTT, Triton X-100, ATP, dATP, dGTP, dCTP, and dTTP; and the enzyme mixed solution G is a mixed solution of a Vvn endonuclease mutan, a T7 endonuclease mutant, and a Taq DNA polymerase.

7. The method according to claim 6, wherein the mixing reaction is carried out under: a reaction temperature of 37°C and a reaction duration of 10 to 30 min, or a reaction temperature of 65°C and a reaction duration of 20 to 30 min.

8. The method according to claim 1, wherein in step S4, the adapter ligation comprises:
mixing and reacting the A-tailed product with a reaction solution H, a reaction solution I, and an enzyme J to obtain an RNA fragment and a DNA fragment; wherein the reaction solution H comprises Tris-HCl, MgCl₂, DTT, ATP, and PEG 8000; the reaction solution I is an adapter X that is an Illumina adapter or an MGISEQ adapter Ad153; and the enzyme J is a T4 DNA ligase.

9. The method according to claim 8, wherein the mixing reaction is carried out under a reaction temperature of 20°C and a reaction duration of 30 min.

10. The method according to claim 8, wherein in step S4, the first purification treatment comprises:
S41, loading the RNA fragment and DNA fragment and 80 µL of magnetic beads into a centrifuge tube, and inverting or vortexing the centrifuge tube to sufficiently mix the RNA fragment and DNA fragment with the magnetic beads;
S42, repeatedly blowing and beating the RNA fragment and DNA fragment with an EP pipette to mix them well with the magnetic beads, and placing the centrifuge tube on a magnetic rack and standing at room temperature;
S43, removing a supernatant after the solution in the centrifuge tube is cleared;
S44, adding 200 µL of freshly prepared 80 v/v% ethanol to the centrifuge tube to rinse the magnetic beads;
S45, repeating steps S42 to S44;
S46, upon centrifuging the centrifuge tube, placing the centrifuge tube on the magnetic rack, and completely removing the supernatant in the centrifuge tube; and
S47, taking out a sample from the centrifuge tube, adding 22 µL of nuclease-removed ultrapure water, gently pipetting, beating and mixing well with a pipette, standing at room temperature for 2 min, then placing the same on the magnetic rack, and after the solution is cleared, obtaining the target RNA fragment and DNA fragment.

11. The method according to claim 1, wherein in step S5, the PCR amplification reaction comprises:
mixing and reacting the target RNA fragment and DNA fragment with a reaction solution K and a reaction solution L to obtain the second-generation sequencing library of RNA and DNA; wherein the reaction solution K comprises a 5*Q5 buffer, dATP, dGTP, dCTP, dTTP, and a Q5 hot-start ultra-fidelity DNA polymerase; and the reaction solution L is an Illumina library amplification primer or an MGISEQ library preparation primer.

12. The method according to claim 11, wherein the PCR amplification reaction comprises the following reaction stages under different reaction temperatures and reaction durations:
1) pre-denaturation stage: under a reaction temperature of 95°C and a reaction duration of 3 min;
2) denaturation stage: under a reaction temperature of 95°C and a reaction duration of 20s;
3) annealing stage: under a reaction temperature of 58°C and a reaction duration of 20s;
4) extension stage: under a reaction temperature of 72°C and a reaction duration of 20s;
5) final extension stage: under a reaction temperature of 72°C and a reaction duration of 5 min; and
6) storage stage: under a reaction temperature of 4°C;
wherein the stages 2) to 4) are separately performed for 10 to 14 cycles; and stages 1) and 5) are separately performed for one cycle.

13. The method according to claim 11, wherein upon completion of the PCR amplification reaction, the method further comprises a second purification treatment on a PCR amplification product:
S51, loading the PCR amplification product and 85 µL of magnetic beads into a centrifuge tube, and inverting or vortexing the centrifuge tube to sufficiently mix the PCR amplification product and the magnetic beads;
S52, repeatedly blowing and beating the PCR amplification product with an EP pipette to mix the amplification product with the magnetic beads sufficiently, and placing the centrifuge tube on a magnetic rack for standing at room temperature;
S53, removing a supernatant after the solution in the centrifuge tube is cleared;
S54, adding 200 µL of freshly prepared 80 v/v% ethanol to the centrifuge tube to rinse the magnetic beads;
S55, repeating steps S52 to S54;
S56, upon centrifuging the centrifuge tube, placing the centrifuge tube on the magnetic rack, and completely removing the supernatant in the centrifuge tube;
S57, taking out a sample from the centrifuge tube, adding 22 µL of nuclease-removed ultrapure water, gently pipetting, beating and mixing well with a pipette, standing at room temperature for 2 min, then placing the same on the magnetic rack, and after the solution is cleared, obtaining the second-generation sequencing library of RNA and DNA.

14. The method according to claim 2, wherein a final concentration of Tris-HCl in the reaction solution A is 300 mM, a final concentration of MgCl₂ in the reaction solution A is 18 mM, a final concentration of KCl in the reaction solution A is 300 mM, a final concentration of DTT in the reaction solution A is 60 mM, a final concentration of dATP in the reaction solution A is 3 mM, a final concentration of dGTP in the reaction solution A is 3 mM, a final concentration of dCTP in the reaction solution A is 3 mM, and a final concentration of dTTP in the reaction solution A is 3 mM.

15. The method according to claim 4, wherein a final concentration of Tris-HCl in the reaction solution D is 100 mM, a final concentration of MgCl₂ in the reaction solution D is 6 mM, a final concentration of KCl in the reaction solution D is 300 mM, a final concentration of DTT in the reaction solution D is 20 mM, a final concentration of NAD+ in the reaction solution D is 208 µM, a final concentration of BSA in the reaction solution D is 400 ug/ml, a final concentration of dATP in the reaction solution D is 4 mM, a final concentration of dGTP in the reaction solution D is 4 mM, a final concentration of dCTP in the reaction solution D is 4 mM, and a final concentration of dTTP in the reaction solution D is 4 mM.

16. The method according to claim 6, wherein a final concentration of Tris-HCl in the reaction solution F is 200 mM, a final concentration of MgCl₂ in the reaction solution F is 100 mM, a final concentration of NaCl in the reaction solution F is 500 mM, a final concentration of DTT in the reaction solution F is 100 mM, a final concentration of ATP in the reaction solution F is 5 mM, a final concentration of dATP in the reaction solution F is 5 mM, a final concentration of dGTP in the reaction solution F is 5 mM, a final concentration of dCTP in the reaction solution F is 5 mM, and a final concentration of dTTP in the reaction solution F is 5 mM.

17. The method according to claim 7, wherein when the reaction temperature is 37°C, the reaction duration is selected according to an expected size of an insert fragment of the second-strand cDNA, wherein the expected size of the insert fragment of the second-strand cDNA is 100 bp, and the reaction duration is 25 to 30 min; the expected size of the insert fragment of the second-strand cDNA is 150 bp, and the reaction duration is 15 to 25 min; and the expected size of the insert fragment of the second-strand cDNA is 200 to 700 bp, and the reaction duration is 10 to 15 min.

18. The method according to claim 8, wherein a final concentration of the Illumina adapter or the MGISEQ adapter Ad153 is 2 µM.

19. The method according to claim 8, wherein a final concentration of Tris-HCl in the reaction solution H is 75 mM, a final concentration of MgCl₂ in the reaction solution H is 15 mM, a final concentration of DTT in the reaction solution H is 15 mM, and a final concentration of ATP in the reaction solution H is 2.75 mM.

20. A second-generation sequencing kit of RNA and DNA prepared by the method as defined in any one of claims 1 to 19.
